# EUROPÄISCHE PATENTSCHRIFT

(11) **EP 0 701 428 B1**
(45) Veröffentlichungstag und Bekanntmachung des Hinweises auf die Patenterteilung: **02.05.1997**
(21) Anmeldenummer: 94918804.9
(22) Anmeldetag: 27.05.1994
(51) Int. Cl.: A61K 7/13

(54) **1,2-NAPHTHOCHINON-HALTIGE MITTEL ZUM FÄRBEN KERATINHALTIGER FASERN**
AGENTS CONTAINING 1,2-NAPTHOQUINONE FOR DYEING KERATIN-CONTAINING FIBRES
AGENTS RENFERMANT DE LA 1,2-NAPHTOQUINONE POUR LA TEINTURE DE FIBRES KERATINIQUES

(30) Priorität: 05.06.1993 DE 4318742
(43) Veröffentlichungstag der Anmeldung: 20.03.1996
(73) Patentinhaber: Henkel Kommanditgesellschaft auf Aktien, 40191 Düsseldorf (DE)
(72) Erfinder: MÖLLER, Hinrich, D-40789 Monheim (DE); HÖFFKES, Horst, D-40595 Düsseldorf (DE)
(86) Internationale Anmeldenummer: EP9401732
(87) Internationale Veröffentlichungsnummer: WO9428862

(56) Entgegenhaltungen:
- EP-A- 0 376 776
- EP-A- 0 460 996
- WO-A-90/10747
- DATABASE WPI Week 7340, Derwent Publications Ltd., London, GB; AN 73-5909U & JP,A,48 048 649 (SUNSTAR)

## Beschreibung

Gegenstand der Erfindung sind Mittel zum Färben keratinhaltiger Fasern, die 1,2-Naphthochinone in Kombination mit Aminosäuren, Oligopeptiden, Anilinderivaten, Phenolderivaten, Pyridinderivaten, Pyrimidinderivaten oder Benzoesäurederivaten enthalten.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Mit Oxidationsfarbstoffen lassen sich zwar intensive Färbungen mit guten Echtheitseigenschaften erzielen, die Entwicklung der Farbe geschieht jedoch unter dem Einfluß von starken Oxidationsmitteln wie z. B. H₂O₂, was häufig Schädigungen der Faser zur Folge hat. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbemittel auf Basis von 1,2-Naphthochinonen, bei denen auf den Einsatz von zusätzlichen Oxidationsmitteln verzichtet werden kann, stellen hier eine Alternative dar. Aus dem Chemical-Abstracts-Referat 40937u, Vol. 80, 1974, ist die Verwendung von 1,2-Naphthochinonsulfonsäure als Haarfärbemittel bekannt. Die europäischen Offenlegungsschriften EP 0 376 776 A2 und EP 0 460 996 A1 offenbaren Haarfärbemittel, die neben 1,2-Naphthochinonen Hydroxy- bzw. Aminoindole enthalten.

Überraschenderweise wurde nun gefunden, daß sich 1,2-Naphthochinone auch in Kombination mit Aminosäuren, Oligopeptiden, Anilinderivaten, Phenolderivaten Pyridin- und Pyrimidinderivaten und Benzoesäurederivaten hervorragend zum Färben von keratinhaltigen Fasern eignen, wobei Ausfärbungen von gelb, orange, braun, violettschwarz, blauschwarz bis zu tiefschwarz erhalten werden.

Als keratinhaltige Fasern kommen dabei z.B. Wolle, Pelze, Felle und menschliche Haare in Betracht. Die unten näher bezeichneten Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril,-, Polyurethan- und Polyesterfasern verwendet werden.

Gegenstand der Erfindung sind Mittel zum Färben von keratinhaltigen Fasern, insbesondere menschlichen Haaren, enthaltend
- ein 1,2-Naphthochinon der Formel I 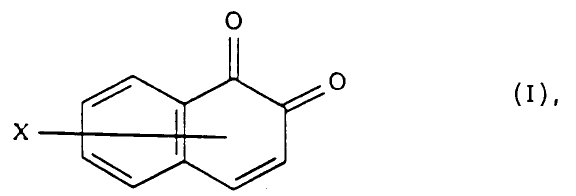 wobei X Wasserstoff, Halogen, eine C₁-C₄-Alkyl-, eine C₁-C₄-Alkoxy-, eine Hydroxy-, eine C₁-C₄-Carboxyalkyl-, eine Sulfo- oder eine Carboxylgruppe darstellt und die Positionen 3 bis 8 einnehmen kann oder deren Salze,
- mindestens eine Aminosäure oder ein aus 2 bis 9 Aminosäuren aufgebautes Oligopeptid
- und einen wasserhaltigen Träger.

Als Aminosäuren kommen alle natürlich vorkommenden und synthetischen Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden, z.B. Tyrosin, Histidin, Phenylalanin, DOPA, Arginin und Tryptophan. Geeignete Oligopeptide sind alle aus natürlich vorkommenden und synthetischen Aminosäuren aufgebauten Oligopeptide. Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mangelprotein enthaltenen Oligopeptide zu nennen.

Ein weiterer Erfindungsgegenstand sind Mittel zum Färben von keratinhaltigen Fasern, insbesondere menschlichen Haaren, enthaltend
- ein 1,2-Naphthochinon der Formel I gemäß Anspruch 1,
- mindestens ein Anilinderivat
- und einen wasserhaltigen Träger.

Geeignete Anilinderivate sind die ein- und mehrfach durch niedere C₁₋₄-Alkyl-, Hydroxyalkyl-, Alkoxy-, Acyloxygruppen, Carboxyl-, Carboxylat-, Sulfo-, Sulfonat-, Amino- oder Aminoalkylgruppen substituierten Anilinderivate, wobei Aminofunktionen durch niederes C₁₋₄-Alkyl, Hydroxyalkyl, Aminoalkyl oder Carboxyalkyl substituiert sein können, z.B. 2-, 3-, 4-Aminophenol, o-, m-, p-Phenylendiamin, 2,5-Diaminotoluol, -phenol, -anisol -phenethol, 2-Chlor-p-phenylendiamin, 4-Methylamino-, 3-, 4-Dimethylamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-6-chlor-, 2-Methyl-5-amino-4-chlor-, 2-Methyl-5-amino-6-chlor-, 5-(2-Hydroxyethylamino)-4-methoxy-2-methyl-, 4-Amino-2-aminomethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexamaminobenzol-hexahydrochlorid, 2,4,6-Triaminoresorcin-trihydrochlorid, 4,5-Diaminobrenzcatechin-sulfat, 4,6-Diaminopyrogallol-dihydrochlorid, 3,5-Diamino-4-hydroxybrenzcatechin-sulfat, 4,4'-Diamino-diphenylamin-sulfat, 4,4'-Diamino-diphenylamin-2-sulfonsäure, 1,8-Bis-(2,5-diaminophenoxy)-3,6-dioxaoctan x 4 HCl, 1,3-Bis-(2,4-diaminophenoxy)-propan x 4 HCl, N,N'-Bis-(2-hydroxyethyl)-p-phenylendiamin x 2 HCl, 2-(2,5-Diaminophenyl)-ethanol x 2 HCl.

Ein weiterer Erfindungsgegenstand sind Mittel zum Färben von keratinhaltigen Fasern, insbesondere menschlichen Haaren, enthaltend
- ein 1,2-Naphthochinon der Formel I gemäß Anspruch 1,
- mindestens ein aliphatisches Amin
- und einen wasserhaltigen Träger.

Geeignete aliphatische Amine sind z.B. 2-Aminoethanol, 2-Methoxy-, 2-Ethoxyethylamin, 2-(2-Aminoethoxy)-ethanol, 2-, 3-Aminopropanol, 2,3-Dihydroxypropylamin, 4-Hydroxypropylamin, 2-Aminopropan-1,3-diol, 2-Amino-2-methylpropanol, 2-Amino-2-methylpropan-1,3-diol, 2-Amino-2-hydroxymethylpropan-1,3-diol, Tetrahydroxypentylamine, Pentahydroxyhexylamine (z.B. Glucamin), Diamine wie 1,2-Diaminoethan, 1,2-, 1,3-Diaminopropan, 1,3-Diamino-2-propanol, 2-(2-Aminoethylamino)-ethylamin, - ethanol, 3-(2-Aminoethylamino)-propylamin, -propanol.

Ein weiterer Erfindungsgegenstand sind Mittel zum Färben von keratinhaltigen Fasern, insbesondere menschlichen Haaren, enthaltend
- ein 1,2-Naphthochinon der Formel I gemäß Anspruch 1,
- mindestens ein Phenolderivat
- und einen wasserhaltigen Träger.

Geeignete Phenolderivate sind z.B. Resorcin, 2-,4-Methylresorcin, Pyrogallol, 1,3,5-, 1,2,4-Trihydroxybenzol, 2,5-Dimethylresorcin.

Ein weiterer Erfindungsgegenstand sind Mittel zum Färben von keratinhaltigen Fasern, insbesondere menschlichen Haaren, enthaltend
- ein 1,2-Naphthochinon der Formel I gemäß Anspruch 1,
- mindestens ein Benzoesäurederivat
- und einen wasserhaltigen Träger.

Geeignete Benzoesäurederivate sind z.B. 2,4-, 3,5-Dihydroxy-, 2,4,6-, 3,4,5-Trihydroxybenzoesäure, 4-,5-Aminosalicylsäure.

Ein weiterer Erfindungsgegenstand sind Mittel zum Färben von keratinhaltigen Fasern, insbesondere menschlichen Haaren, enthaltend
- ein 1,2-Naphthochinon der Formel I gemäß Anspruch 1
- mindestens ein monocyclisches Pyridin- oder Pyrimidinderivat
- und einen wasserhaltigen Träger.

Geeignete monocyclische Pyridin- und Pyrimidinderivate sind z.B. 3,4,5-Triamino-, 2,3,4,5-Tetraaminopyrimidin, 2,4,5-Triamino-3-hydroxypyrimidin, 2,5-Diaminopyridin, 2,6-Dihydroxy-3,4-dimethylpyridin, 3,5-Diamino-2,6-dimethoxypyridin und ihre Salze.

In allen erfindungsgemäßen Färbemitteln können auch mehrere verschiedene 1,2-Naphthochinone der Formel I gemeinsam zum Einsatz kommen. Besonders bevorzugt ist 1,2-Naphthochinon-4-sulfon- bzw. -carbonsäure.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die erfindungsgemäßen Färbemittel in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf dem Haar geeignet sind.

Der wasserhaltige kosmetische Träger enthält üblicherweise Netz- und Emulgiermittel wie anionische, nichtionische oder ampholytische Tenside, z.B. Fettalkoholsulfate, Alkansulfonate, α-Olefinsulfonate, Fettalkoholpolyglykolethersulfate, Alkylglycoside, Ethylenoxidanlagerungsprodukte an Fettalkohole, an Fettsäuren, an Alkylphenole, an Sorbitanfettsäureester, an Fettsäurepartialglyceride und Fettsäurealkanolamide; Verdickungsmittel, z. B. Fettalkohole, Fettsäuren, Paraffinöle, Fettsäureester und andere Fettkomponenten in emulgierter Form; wasserlösliche polymere Verdickungsmittel wie natürliche Gummen, z. B. Gummi arabicum, Karaya-Gummi, Guar-Gummi, Johannisbrotkernmehl, Leinsamengummen und Pektin, biosynthetische Gummen, z.B. Xanthan-Gummi und Dextrane, synthetische Gummen, z. B. Agar-Agar und Algin, Stärke-Fraktionen und Derivate wie Amylose, Amylopektin und Dextrine, modifizierte Cellulosemoleküle, z. B. Methylcellulose, Hydroxyalkylcellulose und Carboxymethylcellulose, Tone wie z. B. Bentonit oder vollsynthetische Hydrokolloide, z.B. Polyvinylalkohol oder Polyvinylpyrrolidon, haarpflegende Zusätze, wie z.B. wasserlösliche kationische Polymere, anionische Polymere, nichtionische Polymere, amphotere oder zwitterionische Polymere, Pantothensäure, Vitamine, Pflanzenextrakte oder Cholesterin, pH-Stellmittel, Komplexbildner und Parfumöle sowie Reduktionsmittel zur Stabilisierung der Inhaltsstoffe, z. B. Ascorbinsäure, schließlich können auch Farbstoffe zum Einfärben der kosmetischen Zubereitungen enthalten sein.

Die 1,2-Naphthochinone der Formel I sowie die Aminosäuren, Oligopeptide, Anilinderivate, aliphatischen Amine, Phenolderivate, Pyridin- oder Pyrimidinderivate bzw. Benzoesäurederivate sind dabei in einer Menge von jeweils 0,3 bis 65, mMol vorzugsweise 6 bis 20 mMol, jeweils bezogen auf 100 g des gesamten Färbemittels, enthalten.

Für das Färbeergebnis kann es vorteilhaft sein, den Färbemitteln Ammonium- oder Metallsalze zuzugeben. Geeignete Metallsalze sind z.B. die Carbonate, Halogenide, Sulfate, Nitrate, Acetate, Lactate, Glykolate, Formiate, Valeriate, Capronate, Tartrate, Citrate, Gluconate, Propionate, Phosphate, Sulfonate und Phosphonate des Kaliums, Natriums, Lithiums, Magnesiums, Calciums, Strontiums, Bariums, Mangans, Eisens, Kobalts, Kupfers, Zinks, Lanthans; bevorzugt sind Lithiumbromid, Calciumbromid, Calciumgluconat, Strontiumchlorid, Strontiumnitrat, Aluminiumchlorid, Aluminiumlactat, Zinkchlorid, Zinksulfat, Magnesiumchlorid, Magnesiumsulfat, Lanthannitrat, Ammoniumcarbonat, -chlorid und -acetat, die im gegebenen Falle in einer Menge von 0,3 bis 65 mMol vorzugsweise 6 bis 20 mMol, bezogen auf 100 g des gesamten Färbemittels, enthalten sind.

Der pH-Wert der gebrauchsfertigen Färbezubereitungen liegt zwischen 2 und 11, vorzugsweise zwischen 5 und 9.

Zum Haarefärben werden die erfindungsgemäßen Färbemittel in Form des wasserhaltigen, kosmetischen Trägers in einer Menge von 100 g auf das Haar aufgebracht, ca. 30 Minuten dort belassen und dann ausgespült oder mit einem handelsüblichen Haarsphampoo ausgewaschen.

Die beiden reaktiven Komponenten (1,2-Naphthochinon der Formel I und Aminosäure, Oligopeptid, Anilinderivat, aliphatisches Amin, Phenolderivat, Pyridin- oder Pyrimidinderivat bzw. Benzoesäurederivat) können entweder gleichzeitig auf das Haar aufgebracht werden oder aber auch nacheinander, wobei es keine Rolle spielt, welche der beiden Komponenten zuerst aufgetragen wird; zwischen dem Auftragen der ersten und der zweiten Komponente können bis zu 30 Minuten Zeitabstand liegen.

### Beispiele

### Herstellung einer Färbelösung:

Es wurde eine Aufschlämmung von 1o mMol eines 1,2-Naphthochinons und 10 mMol der zweiten erfindungsgemäßen Komponente in 100 ml Wasser bereitet. Fakultativ wurden Salze in einer Menge von jeweils 10 mMol zugegeben. Die Aufschlämmung wurde auf Siedetemperatur erhitzt und nach dem Abkühlen filtriert, der pH-Wert wurde anschließend auf 6 bzw. 9 eingestellt (siehe dazu die folgende Tabelle).

In diese Färbelösung wurden bei 35°C 30 Minuten lang zu 90 % ergraute, nicht vorbehandelte Menschenhaare eingebracht. Die jeweiligen Färbetemperaturen, Färbedauern, Farbnuancen und Farbtiefen sind den Tabellen 1 und 2 zu entnehmen. Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **Ausfärbungen mit 1,2-Naphthochinon-4-sulfonsäure** | | | | |
|---|---|---|---|---|
| Aminosäure, Oligopetid, Anilinderivat, aliphatisches Amin, Phenolderivat, Pyridinderivat, Pyrimidinderivat bzw. Benzoesäurederivat | Salze (10 mMol) | pH-Wert | Färbenuance | Farbtiefe |
| --- | NaAc | 6,0 | dunkelbraun | ++(+) |
| 1,2,4,5-Tetraaminobenzol | NaAc | 6,0 | dunkelbraun | +++ |
| 4,4'-Diaminostilben x 2HCl | NaAc | 6,0 | beige | (+) |
| p-Phenylendiamin | NaAc | 6,0 | rotbraun | + |
| 3-Aminomethyl-4-hydroxyanilin | NaAc | 6,0 | dunkelrotbraun | ++(+) |
| 2,4-Dichlor-3-hydroxyanilin | NaAc | 6,0 | gelbbraun | +(+) |
| 2-Methylresorcin | NaAc | 6,0 | gelbbraun | +(+) |
| 2-Chlor-3-hydroxy-4-methylanilin | NaAc | 6,0 | braungeld | + |
| L-Tryptophan | NaAc | 6,0 | orangebraun | + |
| L-Histidin | NaAc | 6,0 | mittelbraun | ++ |
| L-Tyrosin | NaAc | 6,0 | orangebraun | +(+) |
| L-DOPA | NaAc | 6,0 | gelbbraun | +(+) |
| L-Ornithin | NaAc | 6,0 | kupfer | +(+) |
| 1,8-Bis-(2,5-diaminophenoxy)-3,6-dioxaoctan x 4 HCl | NaAc | 9,0 | dunkelgrauviolett | ++ |
| 2,6-Dihydroxy-3,4-dimethylpyridin | NaAc | 6,0 | violettrosa | ++ |
| 4,5,6-Triamino-pyrimidin | NaAc | 6,0 | orange | ++ |

**Tabelle 2**

| **Ausfärbungen mit 1,2-Naphthochinon** | | | | |
|---|---|---|---|---|
| Aminosäure, Oligopetid, Anilinderivat, aliphatisches Amin, Phenolderivat, Pyridinderivat, Pyrimidinderivat bzw. Benzoesäurederivat | Salze (10 mMol) | pH-Wert | Färbenuance | Farbtiefe |
| --- | - | 6 | mittelbraun | ++ |
| p-Toluylendiamin H₂SO₄ | - | 6 | dunkelviolettbraun | +++ |
| 2,4,5,6-Tetraaminopyrimidin H₂SO₄ | - | 6 | braunorange | ++(+) |
| 3,4-Diaminobenzoesäure | - | 6 | orangebraun | ++ |
| 2-(2-Hydroxyethyl)p-phenylendiamin H₂SO₄ | - | 6 | drunkelviolettrot | +++ |
| 1,8-Bis-(2,5-diaminophenoxy)-3,6-dioxaoctan | - | 6 | schwarz | +++ |
| 2-Aminomethyl-3-amino-6-methoxypyridin 2HCl | - | 6 | schwarzbraun | +++ |
| 2-Methylamino-4-aminophenol 2HCl | - | 6 | violettrot | ++(+) |
| 2,6-Dihydroxy-3,4-dimethylpyridin | - | 6 | mittelbraun | ++ |
| 1,3-Bis-(2,4-diaminophenoxy)propan 4HCl | - | 6 | braungrau | ++(+) |
| N,N-Bis-(2-hydroxyethyl)-p-phenylendiamin 2HCl | - | 6 | dunkelgrau | ++(+) |
| 4,4'-Diaminodiphenylamin H₂SO₄ | - | 6 | schwarz | +++ |
| 4,4'-Diaminodiphenylamin-2-sulfonsäure | - | 6 | dunkelbraun | ++ |
| 2,4-Dimethyl-3-ethylpyrrol | - | 6 | Kupfer | ++ |
| 1-(o-Aminophenyl)-pyrrol | - | 6 | Kupfer | ++ |
| Pyrrol | - | 6 | graubraun | +(+) |
| L-Histidin | - | 6 | olivgrün | ++ |
| L-Arginin | - | 6 | hellbraun | + |
| L-Tyrosin | - | 6 | mittelbraun | ++ |
| 3,4-Dihydroxyphenylalanin | - | 6 | gelbbraun | +(+) |
| 1,5-Diaminonaphthalin | - | 6 | rotbraun | ++ |
| 1,8-Diaminonaphthalin | - | 6 | mittelbraun | +(+) |
| 2,3-Diaminonaphthalin | - | 6 | braunorange | ++ |
| 2-Amino-5-naphthol-7-sulfonsäure | - | 6 | violettbraun | ++(+) |

## Patentansprüche

1. Mittel zum Färben keratinhaltiger Fasern, insbesondere menschlichen Haaren, enthaltend
- ein 1,2-Naphthochinon der Formel I 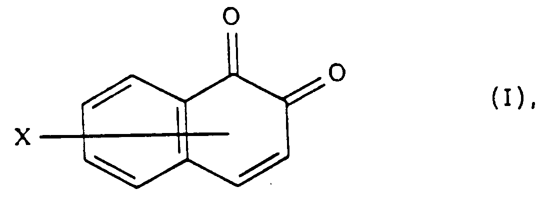 wobei X Wasserstoff, Halogen, eine C₁-C₄-Alkyl-, eine C₁-C₄-Alkoxy-, eine Hydroxy-, eine C₁-C₄-Carboxyalkyl-, eine Sulfo- oder eine Carboxylgruppe darstellt und die Positionen 3 bis 8 einnehmen kann oder deren Salze,
- mindestens eine Aminosäure oder ein aus 2 bis 9 Aminosäuren aufgebautes Oligopeptid
- und einen wasserhaltigen Träger.

2. Mittel zum Färben von keratinhaltigen Fasern, insbesondere menschlichen Haaren, enthaltend
- ein 1,2-Naphthochinon der Formel I gemäß Anspruch 1,
- mindestens ein Anilinderivat
- und einen wasserhaltigen Träger.

3. Mittel zum Färben von keratinhaltigen Fasern, insbesondere menschlichen Haaren, enthaltend
- ein 1,2-Naphthochinon der Formel I gemäß Anspruch 1,
- mindestens ein aliphatisches Amin
- und einen wasserhaltigen Träger.

4. Mittel zum Färben von keratinhaltigen Fasern, insbesondere menschlichen Haaren, enthaltend
- ein 1,2-Naphthochinon der Formel I gemäß Anspruch 1,
- mindestens ein Phenolderivat
- und einen wasserhaltigen Träger.

5. Mittel zum Färben von keratinhaltigen Fasern, insbesondere menschlichen Haaren, enthaltend
- ein 1,2-Naphthochinon der Formel I gemäß Anspruch 1,
- mindestens ein Benzoesäurederivat
- und einen wasserhaltigen Träger.

6. Mittel zum Färben von keratinhaltigen Fasern, insbesondere menschlichen Haaren, enthaltend
- ein 1,2-Naphthochinon der Formel I gemäß Anspruch 1,
- mindestens ein monocyclisches Pyridinderivat
- und einen wasserhaltigen Träger.

7. Mittel nach Anspruch 1 bis 6, dadurch gekennzeichnet, daß das 1,2-Naphthochinon der Formel I 1,2-Naphthochinon-4-sulfon- bzw. -carbonsäure ist.

8. Mittel nach Anspruch 1, dadurch gekennzeichnet, daß ein 1,2-Naphthochinon der Formel I in einer Menge von 0,3 bis 65, mMol vorzugsweise 6 bis 20 mMol, und mindestens eine Aminosäure oder ein aus 2 bis 9 Aminosäuren aufgebautes Oligopeptid in einer Menge von 0,3 bis 65, mMol vorzugsweise 6 bis 20 mMol, jeweils bezogen auf 100 g des gesamten Färbemittels, enthalten sind.

9. Mittel nach Anspruch 2, dadurch gekennzeichnet, daß ein 1,2-Naphthochinon der Formel I in einer Menge von 0,3 bis 65, mMol vorzugsweise 6 bis 20 mMol, und mindestens ein Anilinderivat in einer Menge von 0,3 bis 65, mMol vorzugsweise 6 bis 20 mMol, jeweils bezogen auf 100 g des gesamten Färbemittels, enthalten sind.

10. Mittel nach Anspruch 3, dadurch gekennzeichnet, daß ein 1,2-Naphthochinon der Formel I in einer Menge von 0,3 bis 65, mMol vorzugsweise 6 bis 20 mMol, und mindestens ein aliphatisches Amin in einer Menge von 0,3 bis 65, mMol vorzugsweise 6 bis 20 mMol, jeweils bezogen auf 100 g des gesamten Färbemittels, enthalten sind.

11. Mittel nach Anspruch 4, dadurch gekennzeichnet, daß ein 1,2-Naphthochinon der Formel I in einer Menge von 0,3 bis 65, mMol vorzugsweise 6 bis 20 mMol, und mindestens ein Phenolderivat in einer Menge von 0,3 bis 65, mMol vorzugsweise 6 bis 20 mMol, jeweils bezogen auf 100 g des gesamten Färbemittels, enthalten sind.

12. Mittel nach Anspruch 5, dadurch gekennzeichnet, daß ein 1,2-Naphthochinon der Formel I in einer Menge von 0,3 bis 65, mMol vorzugsweise 6 bis 20 mMol, und mindestens ein Benzoesäurederivat in einer Menge von 0,3 bis 65, mMol vorzugsweise 6 bis 20 mMol, jeweils bezogen auf 100 g des gesamten Färbemittels, enthalten sind.

13. Mittel nach Anspruch 6, dadurch gekennzeichnet, daß ein 1,2-Naphthochinon der Formel I in einer Menge von 0,3 bis 65, mMol vorzugsweise 6 bis 20 mMol, und mindestens ein monocyclisches Pyridin- oder Pyrimidinderivat in einer Menge von 0,3 bis 65, mMol vorzugsweise 6 bis 20 mMol, jeweils bezogen auf 100 g des gesamten Färbemittels, enthalten sind.

## Claims

1. Formulations for colouring keratin-containing fibres, more particularly human hair, containing
- a 1,2-naphthoquinone corresponding to formula I: 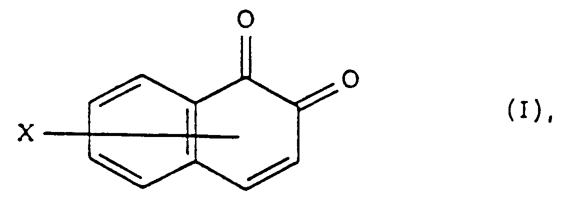 in which X is hydrogen, halogen, a C₁₋₄ alkyl group, a C₁₋₄ alkoxy group, a hydroxy group, a C₁₋₄ carboxyalkyl group, a sulfo group or a carboxyl group and can occupy positions 3 to 8, or salts thereof,
- at least one amino acid or an oligopeptide made up of 2 to 9 amino acids and
- a water-containing carrier.

2. Formulations for colouring keratin-containing fibres, more particularly human hair, containing
- a 1,2-naphthoquinone corresponding to formula I in claim 1,
- at least one aniline derivative and
- a water-containing carrier.

3. Formulations for colouring keratin-containing fibres, more particularly human hair, containing
- a 1,2-naphthoquinone corresponding to formula I in claim 1,
- at least one aliphatic amine and
- a water-containing carrier.

4. Formulations for colouring keratin-containing fibres, more particularly human hair, containing
- a 1,2-naphthoquinone corresponding to formula I in claim 1,
- at least one phenol derivative and
- a water-containing carrier.

5. Formulations for colouring keratin-containing fibres, more particularly human hair, containing
- a 1,2-naphthoquinone corresponding to formula I in claim 1,
- at least one benzoic acid derivative and
- a water-containing carrier.

6. Formulations for colouring keratin-containing fibres, more particularly human hair, containing
- a 1,2-naphthoquinone corresponding to formula I in claim 1,
- at least one monocyclic pyridine derivative and
- a water-containing carrier.

7. Formulations as claimed in claims 1 to 6, characterized in that the 1,2-naphthoquinone corresponding to formula I is 1,2-naphthoquinone-4-sulfonic or carboxylic acid.

8. Formulations as claimed in claim 1, characterized in that a 1,2-naphthoquinone corresponding to formula I is present in a quantity of 0.3 to 65 mmoles and preferably 6 to 20 mmoles and at least one amino acid or an oligopeptide made up of 2 to 9 amino acids is present in a quantity of 0.3 to 65 mmoles and preferably 6 to 20 mmoles, based on 100 g of the colouring formulation as a whole.

9. Formulations as claimed in claim 2, characterized in that a 1,2-naphthoquinone corresponding to formula I is present in a quantity of 0.3 to 65 mmoles and preferably 6 to 20 mmoles and at least one aniline derivative is present in a quantity of 0.3 to 65 mmoles and preferably 6 to 20 mmoles, based on 100 g of the colouring formulation as a whole.

10. Formulations as claimed in claim 3, characterized in that a 1,2-naphthoquinone corresponding to formula I is present in a quantity of 0.3 to 65 mmoles and preferably 6 to 20 mmoles and at least one aliphatic amine is present in a quantity of 0.3 to 65 mmoles and preferably 6 to 20 mmoles, based on 100 g of the colouring formulation as a whole.

11. Formulations as claimed in claim 4, characterized in that a 1,2-naphthoquinone corresponding to formula I is present in a quantity of 0.3 to 65 mmoles and preferably 6 to 20 mmoles and at least one phenol derivative is present in a quantity of 0.3 to 65 mmoles and preferably 6 to 20 mmoles, based on 100 g of the colouring formulation as a whole.

12. Formulations as claimed in claim 5, characterized in that a 1,2-naphthoquinone corresponding to formula I is present in a quantity of 0.3 to 65 mmoles and preferably 6 to 20 mmoles and at least one benzoic acid derivative is present in a quantity of 0.3 to 65 mmoles and preferably 6 to 20 mmoles, based on 100 g of the colouring formulation as a whole.

13. Formulations as claimed in claim 6, characterized in that a 1,2-naphthoquinone corresponding to formula I is present in a quantity of 0.3 to 65 mmoles and preferably 6 to 20 mmoles and at least one monocyclic pyridine or pyrimidine derivative is present in a quantity of 0.3 to 65 mmoles and preferably 6 to 20 mmoles, based on 100 g of the colouring formulation as a whole.

## Revendications

1. Agents pour la teinture de fibres kératiniques, en particulier des cheveux humains, contenant
- une 1,2-naphtoquinone de la formule I 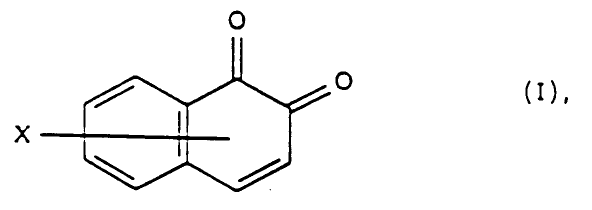 dans laquelle X représente l'hydrogène, un halogène, un groupe alkyle en C₁-C₄, alcoxyle en C₁-C₄, hydroxyle, carboxyalkyle en C₁-C₄ , sulfo ou carboxyle, et peut occuper les positions 3 à 8, ou les sels de ceux-ci,
- au moins un acide aminé ou un oligopeptide constitué de 2 à 9 acides aminés
- et un vecteur aqueux.

2. Agents pour la teinture de fibres kératiniques, en particulier des cheveux humains, contenant
- une 1,2-naphtoquinone de la formule I conformément à la revendication 1,
- au moins un dérivé d'aniline,
- et un vecteur aqueux.

3. Agents pour la teinture de fibres kératiniques, en particulier des cheveux humains, contenant
- une 1,2-naphtoquinone de la formule I conformément à la revendication 1,
- au moins une amine aliphatique,
- et un vecteur aqueux.

4. Agents pour la teinture de fibres kératiniques, en particulier des cheveux humains, contenant
- une 1,2-naphtoquinone de la formule I conformément à la revendication 1,
- au moins un dérivé phénolique,
- et un vecteur aqueux.

5. Agents pour la teinture de fibres kératiniques, en particulier des cheveux humains, contenant
- une 1,2-naphtoquinone de la formule I conformément à la revendication 1,
- au moins un dérivé d'acide benzoïque,
- et un vecteur aqueux.

6. Agents pour la teinture de fibres kératiniques, en particulier des cheveux humains, contenant
- une 1,2-naphtoquinone de la formule I conformément à la revendication 1,
- au moins un dérivé monocyclique de pyridine,
- et un vecteur aqueux.

7. Agents selon les revendications 1 à 6, caractérisés en ce que la naphtoquinone de la formule I est un acide 1,2-naphtoquinone-4-sulfonique ou -carboxylique.

8. Agents selon la revendication 1, caractérisés en ce qu'ils contiennent une 1,2-naphtoquinone de la formule I dans une proportion de 0,3 à 65 mmoles, de préférence de 6 à 20 mmoles, et au moins un acide aminé ou un oligopeptide constitué de 2 à 9 acides aminés, dans une proportion de 0,3 à 65 mmoles, de préférence de 6 à 20 mmoles, dans chaque cas pour 100 g de la totalité de la teinture.

9. Agents selon la revendication 2, caractérisé en ce qu'ils contiennent une 1,2-naphtoquinone de la formule I dans une proportion de 0,3 à 65 mmoles, de préférence de 6 à 20 mmoles et au moins un dérivé d'aniline, dans une proportion de 0,3 à 65 mmoles, de préférence de 6 à 20 mmoles, dans chaque cas pour 100 g de la totalité de la teinture.

10. Agents selon la revendication 3, caractérisés en ce qu'ils contiennent une 1,2-naphtoquinone de la formule I, dans une proportion de 0,3 à 65 mmoles, de préférence de 6 à 20 mmoles et au moins une amine aliphatique, dans une proportion de 0,3 à 65 mmoles, de préférence de 6 à 20 mmoles, dans chaque cas pour 100 g de la totalité de la teinture.

11. Agents selon la revendication 4, caractérisés en ce qu'ils contiennent une 1,2-naphtoquinone de la formule I, dans une proportion de 0,3 à 65 mmoles, de préférence de 6 à 20 mmoles et au moins un dérivé phénolique, dans une proportion de 0,3 à 65 mmoles, de préférence de 6 à 20 mmoles, dans chaque cas pour 100 g de la totalité de la teinture.

12. Agents selon la revendication 5, caractérisés en ce qu'ils contiennent une 1,2-naphtoquinone de la formule I, dans une proportion de 0,3 à 65 mmoles, de préférence de 6 à 20 mmoles et au moins un dérivé d'acide benzoïque, dans une proportion de 0,3 à 65 mmoles, de préférence de 6 à 20 mmoles, dans chaque cas pour 100 g de la totalité de la teinture.

13. Agents selon la revendication 6, caractérisés en ce qu'ils contiennent une 1,2-naphtoquinone de la formule I, dans une proportion de 0,3 à 65 mmoles, de préférence de 6 à 20 mmoles et au moins un dérivé monocyclique de pyridine ou de pyrimidine, dans une proportion de 0,3 à 65 mmoles, de préférence de 6 à 20 mmoles, dans chaque cas pour 100 g de la totalité de la teinture.
